Europäisches Patentamt

(19) European Patent Office

Office européen des brevets

(11) EP 0 559 510 B1

(12) FASCICULE DE BREVET EUROPEEN

(45) Date de publication et mention
de la délivrance du brevet:
24.01.1996 Bulletin 1996/04

(51) Int. Cl.⁶: $C07C\ 7/12$, $C07C\ 7/13$

(21) Numéro de dépôt: 93400413.6

(22) Date de dépôt: 18.02.1993

(54) **Séparation de paraffines aliphatiques par adsorption**

Abtrennung aliphatischer Paraffine durch Adsorption

Separation of aliphatic paraffines by adsorption

(84) Etats contractants désignés:
BE DE GB NL

(30) Priorité: 06.03.1992 FR 9202811

(43) Date de publication de la demande:
08.09.1993 Bulletin 1993/36

(73) Titulaire: INSTITUT FRANCAIS DU PETROLE
F-92502 Rueil-Malmaison (FR)

(72) Inventeurs:
• **Benazzi, Eric**
  **F-78360 Montesson (FR)**
• **Hotier, Gérard**
  **F-92500 Rueil Malmaison (FR)**
• **Basset, Jean-Marie**
  **F-69100 Villeurbanne (FR)**
• **Choplin, Agnès**
  **F-69100 Villeurbanne (FR)**
• **Theolier, Albert**
  **F-69150 Decines (FR)**
• **Nedez, Christophe**
  **F-679190 Saint Fons (FR)**

(56) Documents cités:
WO-A-90/09845        US-A- 3 755 144
US-A- 4 804 802

## Description

L'invention concerne un procédé de séparation de paraffines aliphatiques, lequel procédé utilise au moins un lit d'adsorbant comprenant au moins un solide microporeux greffé, permettant la séparation au moins partielle des paraffines les moins ramifiées et des paraffines les plus ramifiées. Dans une forme préférée du procédé selon l'invention, la séparation entre les paraffines les moins ramifiées et les paraffines les plus ramifiées est totale.

Une des utilisations préférées de l'invention concerne le domaine de l'isomérisation des essences de façon à améliorer leur indice d'octane. En effet, du point de vue de l'indice d'octane, il est souhaitable que les hydrocarbures constituant l'essence soient les plus ramifiés possible. Par exemple, les diméthylbutanes ont un meilleur indice d'octane que les méthylpentanes. Pour augmenter le taux de paraffines polyméthylbranchées comprises dans la charge issue d'une zone d'isomérisation, on peut employer des tamis moléculaires sélectifs quant à la dimension des pores accessibles et recycler à l'isomérisation les paraffines normales mais aussi les paraffines monobranchées. C'est ainsi que le procédé selon l'invention, permettant la séparation au moins partielle des paraffines issues de l'isomérisation, associé éventuellement à un recyclage à l'isomérisation des paraffines normales et/ou monométhylbranchées, permet d'améliorer l'indice d'octane des essences.

Les procédés décrits précédemment impliquent l'isomérisation partielle ou totale des paraffines normales ; ces procédés, décrits dans les brevets US-A-4210771 (Holcombe), US-A-3755144 (Asselin) et US-A-4476345 (Gray), utilisent des zéolithes qui, en raison du diamètre de leurs pores, adsorbent les paraffines normales mais non les produits ramifiés ou cycliques. La zéolithe la plus fréquemment utilisée est la zéolithe dite 5A qui est une zéolithe A dont le cation de substitution est le calcium, le diamètre de ses pores se situant dans la fourchette allant de 3 à 5 Å ; elle adsorbe les paraffines normales mais aucune paraffine ramifiée même monométhylbranchée.

Le brevet US-A-4804802 apporte une amélioration dans la mesure où il décrit l'emploi d'un autre tamis moléculaire qui permet le recyclage à l'isomérisation non seulement des paraffines normales mais aussi des paraffines monométhylbranchées.

Le principe du brevet US-A-4804802 est de traiter la coupe d'hydrocarbures isomérisée sur un lit de tamis moléculaire dont les pores ont un diamètre inférieur ou égal à 4,5 Å et qui n'adsorbe que les paraffines normales, laissant percoler les paraffines monométhylbranchées et bien entendu plus ramifiées. Le second lit de tamis moléculaire est constitué d'une zéolithe dont le diamètre des pores est intermédiaire entre 4,5 et 5,5 Å ; ce lit adsorbe les paraffines monométhylbranchées alors que les paraffines plus ramifiées ne sont pas retenues et constituent la coupe isomérisée à indice d'octane amélioré.

Le premier lit de tamis moléculaire décrit dans ledit brevet peut être un tamis 5A substitué au calcium mais d'autres tamis moléculaires sont utilisables comme des zéolithes R ou des zéolithes T ou même des zéolithes naturelles comme la chabazite ou l'érionite.

Le tamis moléculaire du second lit décrit dans ledit brevet doit avoir une dimension de pores intermédiaire entre celle de la zéolithe 5A calcium et celle de la zéolithe ZSM5, c'est-à-dire que la taille des pores est comprise entre 4,5 Å x 4,5 Å et 5,5 Å x 5,5 Å. La zéolithe recommandée dans le brevet US-A-4804802 est la ferriérite qui, par exemple, adsorbe bien les monométhylpentanes. La ferriérite peut être utilisée sous la forme hydrogène, mais on préfère généralement la forme substituée par un métal alcalin, alcalino-terreux ou un métal de transition. D'autres adsorbants peuvent être utilisés et sont revendiqués dans ledit brevet : ce sont en particulier des aluminophosphates, des silicoaluminophosphates et des borosilicates. Tous ces adsorbants retiennent les paraffines monométhylbranchées et excluent les paraffines diméthylbranchées et les paraffines polyméthylbranchées.

La ferriérite, ainsi que les autres adsorbants dont la taille des pores est comprise entre 4,5 Å x 4,5 Å et 5,5 Å x 5,5 Å, présentent les deux inconvénients majeurs suivants :

- le faible diamètre des pores, nécessaire à l'obtention d'une sélectivité, limite forcément la capacité d'adsorption ainsi que la cinétique d'adsorption et désorption,
- le diamètre des pores, fixé une fois pour toute pour un type d'adsorbant donné, n'est pas absolument optimal et ne permet pas une séparation totale entre les hydrocarbures paraffiniques monométhylbranchés et diméthylbranchés.

De plus, dans le cas de la ferriérite, il est quasiment obligatoire d'un point de vue technique d'utiliser une ferriérite de synthèse présentant beaucoup moins de défauts structuraux que la ferriérite naturelle.

Le procédé selon l'invention consiste en la séparation au moins partielle de paraffines aliphatiques comprises dans une charge hydrocarbonée et contenant de 6 à 14 atomes de carbone par molécule, de préférence de 5 à 6 atomes de carbone par molécule, en un effluent comprenant les paraffines aliphatiques les moins ramifiées et un effluent comprenant les paraffines aliphatiques les plus ramifiées, ledit procédé étant caractérisé par l'utilisation d'au moins un lit d'adsorbant comprenant au moins un solide microporeux greffé. L'utilisation selon l'invention d'au moins un lit d'adsorbant comprenant au moins un solide microporeux permet la rétention au moins partielle sur ledit solide des paraffines les moins ramifiées, c'est-à-dire par exemple des paraffines normales et/ou des paraffines monométhylbranchées, tandis que les paraffines les plus ramifiées, c'est-à-dire par exemple les paraffines monométhylbranchées et/ou les paraffines polyméthylbranchées (c'est-à-dire comportant au moins deux branchements) s'écoulent quasiment librement à travers ledit lit. Les paraffines les moins ramifiées sont récupérées par la suite par balayage à contre-courant avec un éluant.

Ainsi, le procédé selon l'invention peut permettre différentes séparations au moins partielles telles que par exemple les suivantes :

- la séparation au moins partielle des paraffines normales et des autres paraffines dans le cas où la charge comprend des paraffines normales
- la séparation au moins partielle des paraffines monométhylbranchées (et éventuellement des paraffines normales) et des autres paraffines dans le cas où la charge ne comprend pratiquement pas de paraffines normales
- la séparation au moins partielle des paraffines comportant au moins un groupement tertiobutyle et des autres paraffines.

Dans tous les cas, la séparation est au moins partielle, c'est-à-dire qu'elle permet la rétention sur au moins un solide microporeux greffé d'une certaine fraction des paraffines aliphatiques que l'on désire séparer du reste de la charge. Ladite fraction est comprise entre 60 et 100 % en poids, de préférence entre 80 et 100% en poids et de manière préférée elle est égale à 100% en poids ce qui signifie dans ce cas que la rétention est totale.

Le procédé selon l'invention peut ainsi servir à séparer au moins partiellement les paraffines normales des paraffines aliphatiques branchées et les paraffines aliphatiques branchées entre elles selon leur degré de ramification, grâce à la séparation au moyen d'au moins un lit d'adsorbant comprenant au moins un solide microporeux greffé.

Ainsi, des tests d'adsorption compétitive réalisés avec des mélanges d'hydrocarbures paraffiniques aliphatiques normaux, monométhylbranchés et polyméthylbranchés ont montré, soit une adsorption totale pour les hydrocarbures paraffiniques normaux et monométhylbranchés et un rejet total pour les hydrocarbures paraffiniques polyméthylbranchés permettant une séparation totale, soit un facteur de discrimination assez important pour qu'une séparation préparative soit possible.

En effet, les hydrocarbures diffèrent entre eux par leur diamètre cinétique. Ce dernier augmente en fonction du nombre, de la position et de la nature des ramifications, les n-paraffines ayant le plus petit diamètre cinétique. Pour que l'adsorbant puisse avoir un pouvoir de séparation, il faut que la microporosité de l'adsorbant ait des dimensions intermédiaires entre les diamètres cinétiques des différentes paraffines à séparer. Il n'existe pas de variation continue des dimensions de la microporosité des zéolithes connues. Une méthode qui permet de imiter la pénétration des adsorbats à un encombrement prévu consiste à utiliser une zéolithe à larges pores (plus grands que le tamisage souhaité) et à imiter l'ouverture des micropores en greffant des molécules organométalliques variées dont la taille est maîtrisable.

Dans le procédé selon l'invention, nous proposons donc l'emploi de solides microporeux greffés et de préférence de zéolithes greffées et de manière encore plus préférée de mordénites greffées. Ce greffage est réalisé par réaction chimique et conduit à la formation d'un complexe organo-métallique, à la surface des cristallites de la zéolithe. La réaction de greffage est du type suivant :

$$MeR^1 R^2R^3R^4 + \equiv TOH \rightarrow TOMe\, R^1R^2R^3 + R^4H,$$

où :

- T représente un élément choisi dans le groupe formé par Si, Al, Ga, B et Fe, de préférence Si,

- TOH représente un groupement hydroxyle de surface d'une silice, d'une silice-alumine ou d'un solide microporeux tel que par exemple une zéolithe, (silicoaluminate), ou bien un aluminophosphate microporeux, ou un silicoaluminophosphate microporeux, ou un borosilicate microporeux, ou un gallophosphate microporeux.

- Me est un métal ou un élément à caractère métallique. Il est choisi dans le groupe formé par les éléments suivants : les éléments de la colonne VA de la classification périodique des éléments, par exemple l'antimoine et le bismuth, les éléments de la colonne IVA comme le silicium, le germanium, l'étain et le plomb, les éléments de la colonne IIIA comme l'aluminium, le bore, le gallium, l'indium et le thallium, les éléments de la colonne IIA comme le magnésium et les éléments alcalino-terreux et les éléments de la colonne IIB comme le zinc, le cadmium et le mercure,

- $R^i$, i étant égal à 1, 2, 3 ou 4, est un radical organique tel que par exemple le méthyle, l'éthyle, le propyle, l'isopropyle, le cyclohexyle, le phényle. Mais tout autre radical organique connu de l'homme du métier peut également être utilisé.

C'est en grande partie l'encombrement stérique des radicaux organiques qui détermine la limitation de l'ouverture des pores du solide microporeux sur lequel le composé organométallique est greffé.

Le processus de greffage a été décrit dans la demande de brevet EP-A-461171. Nous rappellerons seulement que le réactif est un composé organométallique $MeR^1R^2R^3R^4$, les radicaux $R^1$, $R^2$, $R^3$ et $R^4$ pouvant être de l'hydrogène ou des radicaux organiques semblables ou non. Néanmoins, l'étape de décomposition des groupements alkyles fixés sur le métal décrite dans ladite demande de brevet n'est pas réalisée dans la présente invention : dans le cadre de la présente invention, seule l'étape de greffage est réalisée. Les complexes organométalliques de surface formés doivent être choisis de telle façon qu'ils soient stables thermiquement mais également qu'ils soient stables en présence d'oxygène et/ou de vapeur d'eau.

Un autre facteur important est la capacité d'adsorption de l'adsorbant pour l'hydrocarbure concerné : dans de nombreux cas, le greffage de surface ne modifie pas sensiblement la capacité d'adsorption par rapport à la mordénite non greffée.

De plus le choix judicieux de l'organo-métallique greffé (nature et quantité) et d'autre part des groupements organiques portés par le métal (nature et encombrement stérique) permet de moduler à volonté et de façon quasiment continue la taille de l'entrée des pores du solide microporeux. A titre d'exemple n'importe quel greffage d'un organométallique précédemment cité permet d'exclure les molécules présentant un groupement tertiobutyle, tandis qu'une exclusion totale des molécules diméthylbranchées sur deux carbones voisins s'obtient par exemple par greffage sur la mordénite du tétracyclohexyl étain.

Enfin, une des utilisations préférées du procédé selon l'invention est l'utilisation, pour augmenter le taux de paraffines polyméthylbranchées issues d'une charge comprenant principalement, c'est-à-dire au moins 95%, des paraffines aliphatiques contenant de 5 à 6 atomes de carbone par molécule, de préférence, d'une charge issue d'une zone d'isomérisation. En effet , on peut employer au moins un solide microporeux permettant de prélever et éventuellement de recycler à l'isomérisation les paraffines normales mais aussi les paraffines monométhylbranchées. Ainsi, dans le cas où l'on traite une charge comprenant principalement des paraffines aliphatiques contenant de 5 à 6 atomes de carbone par molécule, de préférence une charge issue d'une zone d'isomérisation, il est possible de procéder selon l'invention de la façon suivante :

- au moins un lit d'adsorbant permet de séparer la charge en un premier effluent comprenant principalement les paraffines normales et en un deuxième effluent, puis,

- au moins un lit d'adsorbant, sur lequel passe le deuxième effluent comprenant au moins un solide microporeux greffé, permet l'obtention d'un troisième effluent comprenant principalement les paraffines monométhylbranchées et d'un quatrième effluent comprenant principalement les paraffines polyméthylbranchées.

Le premier effluent ainsi que le troisième effluent sont avantageusement recyclés à l'isomérisation dans le cas d'une charge issue d'une zone d'isomérisation.

Les exemples suivants illustrent l'invention.

EXEMPLE 1 (selon l'invention)

On détermine les capacités d'adsorption des échantillons considérés pour les hydrocarbures suivants représentant différents types d'hydrocarbures : le n-hexane, le 2-méthylpentane, le 2,3-diméthylbutane, l'isooctane (2,2,4-triméthylpentane).

L'adsorbant testé est une mordénite dont le rapport Si/Al est de 10 : un essai "à blanc" montre que cet adsorbant n'est pas sélectif pour les hydrocarbures cités et présente une capacité d'adsorption d'environ 12 $cm^3/100$ g.

Après traitement par le tétrabutyl étain, $SnBu_4$, la dimension de l'entrée des pores de l'adsorbant est réduite par des groupes $\equiv Si-O-SnBu_3$. Les capacités d'adsorption des hydrocarbures cités dans l'ordre donné ci-dessus deviennent 7,5-5,4-4,6-0,0 $cm^3/100$ g.

Une mordénite possédant un rapport Si/Al de 40 et traitée par le monohydrure de tributyl étain, $SnBu_3H$, conduit aux résultats suivants pour les capacités d'adsorption : 6,2-4-3,2-0,0 $cm^3/100$ g.

Ceci démontre l'existence d'une discrimination assez importante entre un hydrocarbure monométhylbranché et un hydrocarbure diméthylbranché pour lequel les deux branchements sont consécutifs. De plus, il y a exclusion de l'hydrocarbure triméthylbranché. Enfin, le rapport Si/Al de la mordénite utilisée semble sans influence.

EXEMPLE 2 (selon l'invention)

On reprend les déterminations de l'exemple 1 en mesurant les capacités d'adsorption pour les mêmes hydrocarbures.

Dans un premier cas, on utilise une mordénite, de rapport Si/Al de 10, traitée au tétraméthyl étain qui conduit à la formation d'entités $\equiv Si-O-SnMe_3$ à l'entrée des pores de la mordénite. Dans ce cas, les capacités d'adsorption sont respectivement de 9,5-7,5-4,5-0,0 $cm^3/100$ g, ce qui montre une bonne sélectivité d'adsorption entre l'hydrocarbure monosubstitué et l'hydrocarbure disubstitué avec conservation d'une capacité d'adsorption raisonnable.

Dans un second cas, on utilise une mordénite, de rapport Si/Al de 10, traitée au tétracyclohexyl étain ($SnCy_4$), qui conduit à la formation d'entités $\equiv Si-OSnCy$ greffées à l'entrée des pores de la mordénite. Dans ce cas, il y a adsorption du 2-méthylpentane et rejet total du 2,3-diméthylbutane : c'est un exemple de sélectivité parfaite d'adsorption entre un hydrocarbure monométhylbranché et un hydrocarbure diméthylbranché pour lequel les deux branchements sont consécutifs.

EXEMPLE 3 (comparatif)

Comparaison entre la ferriérite et la mordénite traitée au tétracyclohexyl étain.

Dans cet exemple deux lits d'adsorbants sont utilisés pour traiter un mélange équimolaire de 2-méthylpentane et de 2,3-diméthylbutane. Le premier est constitué de ferriérite H, le second de mordénite traitée au tétracyclohexyl étain. On détermine dans chaque cas la quantité d'adsorbant nécessaire à l'obtention de 1 gramme de 2,3-diméthylbutane pur. Dans le cas de la ferriérite, il faut utiliser 30 g de lit alors que dans le cas de la mordénite greffée 5 g de lit sont suffisants. Cet exemple montre une sélectivité très supérieure de la mordénite greffée.

EXEMPLE 4 (comparatif)

Comparaison entre la ferriérite et la mordénite traitée au tétraméthyl étain.

On répète un essai semblable à celui de l'exemple 3 avec un mélange équimolaire de n-hexane, 3-méthylpentane et 2,3-diméthylbutane, les deux lits d'adsorbants sont constitués de ferriérite H et de mordénite traitée au tétraméthyl étain. On détermine la quantité de lit à utiliser pour produire 1 gramme d'effluent ne contenant pas de n-hexane. Dans le cas de la ferriérite, il faut utiliser 18 g de lit alors que dans le cas de la mordénite greffée il ne faut utiliser que 10 g de lit. Cet exemple montre que la capacité d'adsorption de la mordénite greffée est supérieure à celle de la ferriérite.

**Revendications**

1. Procédé de séparation au moins partielle de paraffines aliphatiques comprises dans une charge hydrocarbonée et contenant de 6 à 14 atomes de carbone par molécule en au moins un effluent comprenant les paraffines les moins ramifiées et au moins un effluent comprenant les paraffines les plus ramifiées, ledit procédé étant caractérisé par l'utilisation d'au moins un lit d'adsorbant comprenant au moins un solide microporeux greffé.

2. Procédé selon la revendication 1 dans lequel ledit solide est une zéolithe greffée.

3. Procédé selon l'une des revendications 1 ou 2 dans lequel ledit solide est une mordénite greffée.

4. Porcédé selon l'une des revendications 1 à 3 dans lequel le greffage a été effectué par réaction chimique d'un composé organo-métallique sur le solide, ledit composé ayant pour formule $MeR^1R^2R^3R^4$, Me étant un élément choisi dans le groupe formé par les éléments des groupes IIA, IIB, IIIA, IVA et VA de la classification périodique des éléments et $R^i$, i étant égal à 1, 2, 3 ou 4, étant l'hydrogène ou un radical organique.

5. Procédé selon la revendication 4 dans lequel l'élément Me est l'étain.

6. Utilisation du procédé selon l'une des revendications 1 à 5 dans lequel la charge comprend principalement des paraffines aliphatiques contenant de 5 à 6 atomes de carbone par molécule et est traitée comme suit :

- au moins un lit d'adsorbant permet de séparer la charge en premier effluent comprenant principalement les paraffines normales et en un deuxième effluent , puis,

- au moins un lit d'adsorbant, sur lequel passe le deuxième effluent comprenant au moins un solide microporeux greffé, permet l'obtention d'un troisième effluent comprenant principalement les paraffines monométhylbranchées et d'un quatrième effluent comprenant principalement les paraffines polyméthylbranchées.

7. Utilisation selon la revendication 6 dans laquelle on recycle le premier et le troisième effluent à une zone d'isomérisation.

8. Utilisation selon l'une des revendications 1 à 7 dans laquelle la charge est issue d'une zone d'isomérisation.

**Claims**

1. Process for the at least partial separation of aliphatic paraffins in a hydrocarbon charge containing 6 to 14 carbon atoms per molecule into at least one effluent comprising the least branched paraffins and at least one effluent comprising the most branched paraffins, said process being characterized by the use of at least one adsorbent bed comprising at least one grafted microporous solid.

2. Process according to claim 1, wherein the solid is a grafted zeolite.

3. Process according to either of the claims 1 and 2, wherein said solid is a grafted mordenite.

4. Process according to any one of the claims 1 to 3, wherein grafting takes place by the chemical reaction of a metalorganic compound on the solid, said compound having the formula $MeR^1R^2R^3R^4$, Me being an element chosen from within the group formed by elements of groups IIA, IIB, IIIA, IVA and VA of the periodic classification of elements and $R^i$, i being equal to 1,2,3 or 4, being hydrogen or an organic radical.

5. Process according to claim 4, wherein the element Me is tin.

6. Use of the process according to any one of the claims 1 to 5, wherein the charge mainly comprises aliphatic paraffins containing 5 to 6 carbon atoms per molecule and is treated in the following way:

at least one adsorbent bed makes it possible to separate the charge into a first effluent mainly comprising normal paraffins and a second effluent and at least one adsorbent bed, onto which is passed the second effluent comprising at least one grafted microporous solid, making it possible to obtain a third effluent mainly comprising the monomethyl branched paraffins and a fourth effluent mainly comprising the polymethyl branched paraffins.

7. Use according to claim 6, wherein the first and third effluents are recycled to an isomerization zone.

8. Use according to any one of the claims 1 to 7, wherein the charge is obtained from an isomerization zone.

## Patentansprüche

1. Verfahren zur wenigstens teilweisen Trennung von aliphatischen Paraffinen, die in einer Kohlenwasserstoffcharge umfaßt sind und 6 bis 14 Kohlenstoffatome pro Molekül enthalten, in wenigstens einen Abstrom, welcher die am wenigsten verzweigten Paraffine umfaßt, und wenigstens einen Abstrom, welcher die am meisten verzweigten Paraffine umfaßt, wobei das Verfahren durch die Verwendung von wenigstens einem Adsorptionsbett gekennzeichnet ist, welches wenigstens einen veredelten mikroporösen Feststoff umfaßt.

2. Verfahren nach Anspruch 1, bei welchem der Feststoff ein veredelter Zeolith ist.

3. Verfahren nach einem der Ansprüche 1 oder 2, bei welchem der Feststoff ein veredelter Mordenit ist.

4. Verfahren nach einem der Ansprüche 1 bis 3, bei welchem die Veredelung durch eine chemische Reaktion einer metallorganischen Verbindung an dem Feststoff durchgeführt worden ist, wobei die Verbindung die Formel $MeR^1R^2R^3R^4$ aufweist, wobei Me ein Element ist, das aus der Gruppe ausgewählt ist, welche durch die Elemente der Gruppen IIA, IIB, IIIA, IVA und VA des Periodensystems gebildet ist, und wobei $R^i$ mit i gleich 1, 2, 3 oder 4 Wasserstoff oder ein organischer Rest ist.

5. Verfahren nach Anspruch 4, bei welchem das Element Me Zinn ist.

6. Verwendung des Verfahrens nach einem der Ansprüche 1 bis 5, bei welcher die Charge hauptsächlich aliphatische Paraffine, die 5 bis 6 Kohlenstoffatome pro Molekül enthalten, umfaßt und wie folgt behandelt wird:

- wenigstens ein Adsorptionsbett gestattet es, die Charge in einen ersten Abstrom, welcher hauptsächlich normale Paraffine umfaßt, und in einen zweiten Abstrom zu trennen, dann

- gestattet wenigstens ein Adsorptionsbett, über das der zweite Abstrom geführt wird, welcher wenigstens einen veredelten mikroporösen Feststoff umfaßt, den Erhalt eines dritten Abstromes, welcher hauptsächlich monomethylverzweigte Paraffine umfaßt, und eines vierten Abstromes, welcher hauptsächlich polymethylverzweigte Paraffine umfaßt.

7. Verwendung nach Anspruch 6, bei welcher man den ersten und den dritten Abstrom in eine Zone zur Isomerisierung zurückführt.

8. Verwendung nach einem der Ansprüche 1 bis 7, bei welcher die Charge aus einer Zone zur Isomerisierung stammt.